# EUROPEAN PATENT APPLICATION

(11) **EP 4 089 099 A1**
(43) Date of publication of application: **16.11.2022**
(21) Application number: 21738639.0
(22) Date of filing: 07.01.2021
(51) Int. Cl.: C07H 21/04

(54) **NUCLEIC ACID SYNTHESIS METHOD USING SEGMENT-TYPE AMIDITE**

(30) Priority: 08.01.2020 US 202062958351 P
(71) Applicant: Nitto Denko Corporation, Ibaraki-shi, Osaka 567-8680 (JP); NITTO DENKO Avecia Inc., Milford, MA 01757 (US)
(72) Inventor: IIDA, Tomoyoshi, Ibaraki-shi, Osaka 567-8680 (JP); IWAMOTO, Masafumi, Ibaraki-shi, Osaka 567-8680 (JP); SAKAMOTO, Kota, Ibaraki-shi, Osaka 567-8680 (JP); PAREDES, Eduardo, Milford, Massachusetts 01757 (US)
(74) Representative: Hoffmann Eitle
(86) International application number: PCT/JP2021/000273
(87) International publication number: WO 2021/141072

(57) **Abstract**

The purpose of the present invention is to provide a method for synthesizing an oligonucleotide by using a segment-type amidite. An oligonucleotide production method comprising at least one coupling step for coupling a nucleoside phosphoramidite with a thiol group or a hydroxyl group at the 3' or 5' of a nucleoside or a nucleotide in the presence of an activator, wherein, in the at least one coupling step, said nucleoside phosphoramidite has (a) two or more nucleoside portions or (b) at least one nucleoside portion and a linker portion, and said activator has a structure represented by formula (1) (in formula (1), X represents an organic base) or by formula (2) (in formula (2), R¹ and R² are each independently selected from the group consisting of H, straight chain or branched chain C₁₋₇ alkyl groups and optionally substituted aromatic groups).

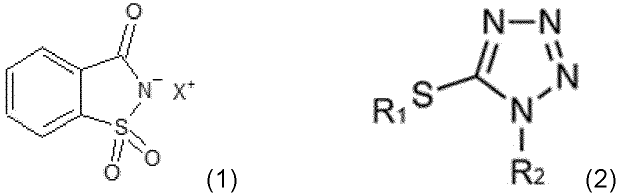

## Description

### [Technical Field]

The present invention relates to a method for synthesizing an oligonucleotide using a segment-type amidite.

### [Background Art]

Phosphoramidite method is widely used in the chemical synthesis of nucleic acids such as DNA oligonucleotides and RNA oligonucleotides. In this method, oligonucleotides having a desired sequence are typically synthesized by sequentially adding appropriately protected nucleosides, one at a time. However, the reaction does not occur with a probability of 100%, and the added nucleosides may be degraded during the synthesis reaction, so that oligonucleotide (N-n)mers with a length 1 or more (n) shorter than the desired length (N) can inevitably be produced. After the synthesis, oligonucleotides are purified by chromatography or the like; an oligonucleotide (N)mer having a desired length (N) and an oligonucleotide (N-1)mer having a length one nucleotide shorter than the desired length have similar chromatographic mobility, and therefore, the presence of the (N-1)mer has a great influence on the purification efficiency of oligonucleotides and the purity of purified products.

In Patent Documents 1 and 2 and Non-Patent Document 1, as a method for suppressing the production of oligonucleotide (N-1)mers, methods in which a nucleoside phosphoramidite having two or three nucleoside moieties (segment-type amidite) is used in the synthesis have been described; however, those methods were not sufficiently efficient.

Also, Patent Document 3 describes a saccharin derivative as an activator that can be used more safely than 1H-tetrazole in the synthesis of oligonucleotides. However, in Patent Document 3, the performance of the saccharin derivative as an activator has not been sufficiently investigated. Furthermore, In Patent Document 4, imidazolium salts and benzimidazolium salts, that are activators containing at least one pyridinium salt and at least one substituted imidazole, are described as activators in place of 1H-tetrazole. However, Patent Document 4 does not describe the use of a salt with saccharin as an activator.

### [Citation List]

### [Patent Document]

[Patent Document 1] WO 02/20543
[Patent Document 2] JP No. 2017-514479
[Patent Document 3] US Pat. No. 7,501,505
[Patent Document 4] US Pat. No. 6,642,373

### [Non-Patent Document]

[Non-Patent Document 1] RNA synthesis via dimer and trimer phosphoramidite block coupling [Tetrahedron Letters 52 (2011) 2575-2578]

### [Summary of the Invention]

### [Problems to Be Solved by the Invention]

The present invention aims to provide a method for synthesizing an oligonucleotide using a segment-type amidite.

### [Means for Solving the Problems]

The inventors of the present invention have earnestly researched a method for synthesizing an oligonucleotide using a segment-type amidite, and found a useful activator in the method. As a result of further research based on such finding, the present inventors have completed the present invention.

That is, the present invention relates to the following.
[1] A method for producing an oligonucleotide, comprising performing one or more coupling steps of binding a nucleoside phosphoramidite to a 3' or 5' hydroxyl or thiol group of a nucleotide or nucleoside in the presence of an activator,
   wherein in at least one coupling step, the nucleoside phosphoramidite is
      (a) a nucleoside phosphoramidite having two or more nucleoside moieties, or
      (b) a nucleoside phosphoramidite having one or more nucleoside moieties and a linker moiety, and
   the activator has a structure represented by the following formula: 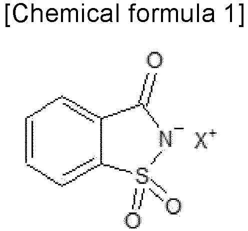
   wherein X is an organic base,
   or by the following formula: 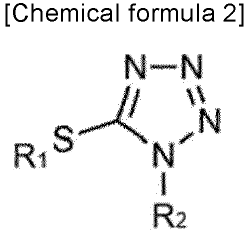
   wherein R¹ and R² are each independently selected from the group consisting of H, linear or branched C₁₋₇ alkyl groups, and aromatic groups which may be optionally substituted.
[2] The method according to [1], wherein
   X is N-methylimidazole, pyridine or 3-methylpyridine,
   R¹ is H, CₙH₂ₙ₊₁ or a benzyl group,
   R² is H, CH₃, or C₆H₅, and
   n is 1, 2 or 3.
[3] The method according to [1] or [2], wherein the activator is 5-mercapto-1-methyltetrazole (1-Me-MCT), 5-mercapto-1-phenyltetrazole (1-Ph-MCT), saccharin 1-methylimidazole (SMI), or 5-ethylthio-1H-tetrazole (ETT).
[4] The method according to any one of [1] to [3], wherein in at least the last one of the coupling steps performed two or more times, the nucleoside phosphoramidite is
   (a) a nucleoside phosphoramidite having two or more nucleoside moieties, or
   (b) a nucleoside phosphoramidite having one or more nucleoside moieties and a linker moiety.
[5] The method according any one of [1] to [4], wherein in at least the last one of the coupling steps performed two or more times,
   the nucleoside phosphoramidite is a nucleoside phosphoramidite having three nucleoside moieties.
[6] The method according to any one of [1] to [5], wherein in at least one of the coupling steps performed two or more times,
   the nucleoside phosphoramidite is a nucleoside phosphoramidite having one nucleoside moiety.
[7] The method according to any one of [1] to [6], wherein only in the last one of the coupling steps performed two or more times,
   the nucleoside phosphoramidite is
      (a) a nucleoside phosphoramidite having three nucleoside moieties, or
      (b) a nucleoside phosphoramidite having one or more nucleoside moieties and a linker moiety, and
   in other coupling steps,
   the nucleoside phosphoramidite is a nucleoside phosphoramidite having one nucleoside moiety.
[8] The method according to any one of [1] to [7], wherein the nucleoside phosphoramidite having two or more nucleoside moieties is a nucleoside phosphoramidite represented by the following formula (I): [Chemical formula 3] 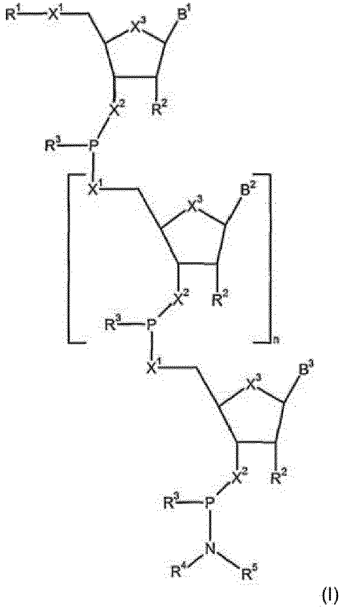 wherein,
   X¹ is each independently -O- or -S-;
   X² is each independently -O- or -S-;
   X³ is each independently -O-, -S-, -CH₂- or -(CH₂)₂-;
   R¹ is a protecting group;
   R² is each independently -H, -NHR⁶, halogen, -CN, -CF₃, or a hydroxyl group protected by an acyl-based protecting group, an ether-based protecting group or a silyl-based protecting group;
   R³ is each independently -OCH₂CH₂CN, -SCH₂CH₂CN, a substituted or unsubstituted aliphatic group, -OR⁷ or -SR⁷;
   R⁴ and R⁵ are each independently a substituted or unsubstituted aliphatic group, a substituted or unsubstituted aromatic group, a substituted or unsubstituted aralkyl group; or
   R⁴ and R⁵ taken together with nitrogen to which they are bound form a heterocycloalkyl group or a heteroaromatic group;
   R⁶ is each independently -H, a substituted or unsubstituted aliphatic group, a substituted or unsubstituted aromatic group, a substituted or unsubstituted aralkyl group, or a protecting group;
   R⁷ is each independently a substituted or unsubstituted aliphatic group, a substituted or unsubstituted aromatic group, or a substituted or unsubstituted aralkyl group;
   8¹, B² and B³ are each independently -H, or a protected or unprotected base; and
   n is 0 or a positive integer;
   or a stereoisomer thereof.
[9] The method according to [8], wherein n is 0 or 1.
[10] The method according to [8] or [9], wherein R² is -H.
[11] The method according to any one of [8] to [10], wherein R³ is - OCH₂CH₂CN.
[12] The method according to any one of [1] to [11], wherein the nucleoside phosphoramidite having one or more nucleoside moieties and a linker moiety is a nucleoside phosphoramidite represented by the following formula (II): 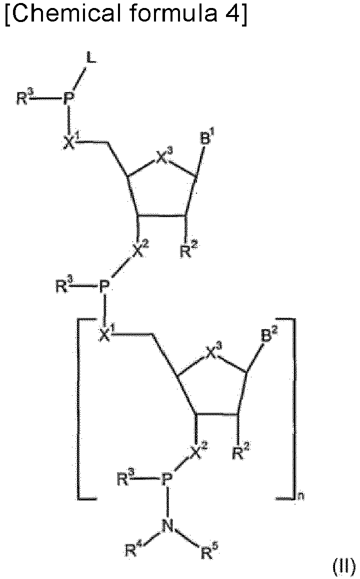 wherein,
   X¹ is each independently -O- or -S-;
   X² is each independently -O- or -S-;
   X³ is each independently -O-, -S-, -CH₂- or -(CH₂)₂-;
   L is a linker;
   R² is each independently -H, -NHR⁶, halogen, -CN, -CF₃, or a hydroxyl group protected by an acyl-based protecting group, an ether-based protecting group or a silyl-based protecting group;
   R³ is each independently -OCH₂CH₂CN, -SCH₂CH₂CN, a substituted or unsubstituted aliphatic group, -OR⁷ or -SR⁷;
   R⁴ and R⁵ are each independently a substituted or unsubstituted aliphatic group, a substituted or unsubstituted aromatic group, a substituted or unsubstituted aralkyl group; or
   R⁴ and R⁵ taken together with nitrogen to which they are bound form a heterocycloalkyl group or a heteroaromatic group;
   R⁶ is each independently -H, a substituted or unsubstituted aliphatic group, a substituted or unsubstituted aromatic group, a substituted or unsubstituted aralkyl group, or a protecting group;
   R⁷ is each independently a substituted or unsubstituted aliphatic group, a substituted or unsubstituted aromatic group, or a substituted or unsubstituted aralkyl group;
   B¹ and B² are each independently -H, or a protected or unprotected base; and n is 0 or a positive integer;
   or a stereoisomer thereof.
[13] A method for producing an oligonucleotide, comprising performing one or more coupling steps of binding a nucleoside phosphoramidite to a 3' or 5' hydroxyl or thiol group of a nucleotide or nucleoside in the presence of an activator,
   wherein in at least one coupling step, the nucleoside phosphoramidite is
      (a) a nucleoside phosphoramidite having two or three nucleoside moieties, or
      (b) a nucleoside phosphoramidite having one or more nucleoside moieties and a linker moiety, and
   the activator has a structure represented by the following formula: 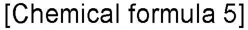 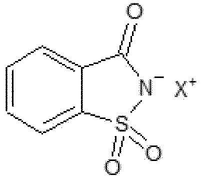
   wherein X is an organic base,
   or by the following formula: 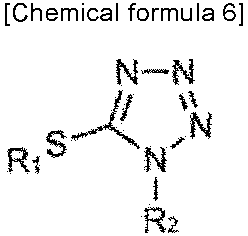
   wherein R¹ and R² are each independently selected from the group consisting of H, linear or branched C₁₋₇ alkyl groups, and aromatic groups which may be optionally substituted.
[14] The method according to [13], wherein
   X is N-methylimidazole, pyridine or 3-methylpyridine,
   R¹ is H, CₙH₂ₙ₊₁ or a benzyl group,
   R² is H, CH₃, or C₆H₅, and
   n is 1, 2 or 3.
[15] A method for producing an oligonucleotide, comprising performing one or more coupling steps of binding a nucleoside phosphoramidite to a 3' or 5' hydroxyl or thiol group of a nucleotide or nucleoside in the presence of an activator,
   wherein in at least one coupling step, the nucleoside phosphoramidite is
      (a) a nucleoside phosphoramidite having two or more nucleoside moieties, or
      (b) a nucleoside phosphoramidite having one or more nucleoside moieties and a linker moiety, and
   the HOMO energy (a.u.) of the activator is -0.21407 to -0.16858 in acetonitrile, and
   the orbital coefficient of the activator is 0.31531 to 0.59405 in acetonitrile.
[16] The method according to [15], wherein the pKa of the activator is 3.65 to 7 in water.
[17] A method for producing an oligonucleotide, comprising performing one or more coupling steps of binding a nucleoside phosphoramidite to a 3' or 5' hydroxyl or thiol group of a nucleotide or nucleoside in the presence of an activator,
   wherein in at least one coupling step, the nucleoside phosphoramidite is
      (a) a nucleoside phosphoramidite having two or three nucleoside moieties, or
      (b) a nucleoside phosphoramidite having one or more nucleoside moieties and a linker moiety, and
   the HOMO energy (a.u.) of the activator is -0.22024 to -0.16858 in acetonitrile, and
   the orbital coefficient of the activator is 0.31531 to 0.59405 in acetonitrile.
[18] The method according to [17], wherein the pKa of the activator is 3.65 to 7 in water.
[19] A method for producing an oligonucleotide, comprising performing one or more coupling steps of binding a nucleoside phosphoramidite to a 3' or 5' hydroxyl or thiol group of a nucleotide or nucleoside in the presence of an activator,
   wherein in at least one coupling step, the nucleoside phosphoramidite is
      (a) a nucleoside phosphoramidite having two or three nucleoside moieties, or
      (b) a nucleoside phosphoramidite having one or more nucleoside moieties and a linker moiety, and
   the pKa of the activator is 3.65 to 7 in water.
[20] A method for producing an oligonucleotide, comprising performing one or more coupling steps of binding a nucleoside phosphoramidite to a 3' or 5' hydroxyl or thiol group of a nucleotide or nucleoside in the presence of an activator,
   wherein in at least one coupling step, the nucleoside phosphoramidite is
      (a) a nucleoside phosphoramidite having two or more nucleoside moieties, or
      (b) a nucleoside phosphoramidite having one or more nucleoside moieties and a linker moiety, and
   the pKa of the activator is 4.3 to 7 in water.

### [Advantageous Effects of the Invention]

By using the activator of the present invention, an oligonucleotide can be synthesized with high efficiency using a nucleoside phosphoramidite having two or more nucleoside moieties, that is, a segment-type amidite, or a nucleoside phosphoramidite having one or more nucleoside moieties and a linker moiety. In addition, by using a nucleoside phosphoramidite having two or more nucleoside moieties, that is, a segment-type amidite, in the last one of the coupling steps, production of (N-1)mers having a chromatographic mobility close to that of the desired oligonucleotide which are difficult to be removed by purification can be suppressed. Furthermore, the use of the activator of the present invention as an activator also contributes to the suppression of the degradation of segment-type amidites during synthetic processes.

### [Embodiments for Carrying out the Invention]

Hereinafter, the present invention will be described in detail.

Unless defined otherwise herein, all technical and scientific terms used herein have the same meaning as commonly understood by one of ordinary skill in the art. All patents, applications and other publications or information referenced herein are hereby incorporated by reference in their entirety. In the case of inconsistency between the publications referred to in this specification and the description in this specification, the description in this specification shall prevail.

The present invention, in one aspect, relates to a method for producing an oligonucleotide, which comprises performing one or more coupling steps of binding a nucleoside phosphoramidite to a nucleotide or nucleoside having an unsubstituted hydroxyl group or an unsubstituted thiol group in the presence of an activator.

In the present invention, the oligonucleotide is produced using a socalled phosphoramidite method, wherein a nucleotide is added by a condensation reaction between a nucleoside phosphoramidite and a nucleoside in a solution or on a solid support, in the presence of a suitable activating agent.

In the present invention, an oligonucleotide refers to a compound having a structure in which a base, a sugar and a phosphoric acid are linked by a phosphodiester bond, and it includes naturally occurring oligonucleotides such as 2'-deoxyribonucleic acid (hereinafter, "DNA") and ribonucleic acid (hereinafter "RNA"), and nucleic acids containing a modified sugar moiety, a modified phosphate moiety, or a modified nucleobase. Modifications to the sugar moiety include replacing the ribose ring with a hexose, cyclopentyl, or cyclohexyl ring. Alternatively, the D-ribose ring of a naturally occurring nucleic acid may be replaced with the L-ribose ring, or the β-anomer of a naturally occurring nucleic acid may be replaced with the α-anomer. Oligonucleotides may also comprise one or more abasic moieties. Modified phosphate moieties include phosphorothioates, phosphorodithioates, methylphosphonates, and methyl phosphate. Such nucleic acid analogs are known to those of skill in the art. Oligonucleotides comprising a mixture of two or more of the above mixtures can be produced from, for example, a mixture of deoxyribo- and ribonucleoside, in particular a mixture of deoxyribonucleoside and 2'-O-substituted ribonucleoside such as 2'-O-methyl ribonucleoside or 2'-O-methoxyethyl ribonucleoside. Examples of oligonucleotides comprising a mixture of nucleosides include ribozymes.

In the present invention, a nucleoside phosphoramidite (segment-type amidite) refers to a nucleoside derivatized with an amidite. Amiditation can be carried out, for example, by reacting an appropriately protected nucleoside with 2-cyanoethyl-N,N,N',N'-tetraisopropylphosphorodiamidite using 1H-tetrazole as an activator.

In the present invention, the nucleoside refers to a compound in which a base and a sugar are bound, and it may be a naturally occurring nucleoside such as adenosine, thymidine, guanosine, cytidine, and uridine, or a modified nucleoside. The base may be a naturally occurring base such as adenine, guanine, cytosine, thymine, and uracil, or a modified base. The sugar moiety of a nucleoside may be naturally occurring deoxyribose or ribose, and it may have the D-configuration or the L-configuration.

In the present invention, the activator is used to react a nucleoside phosphoramidite with a nucleotide or nucleoside, and is also called an activating agent or a coupling agent.

In one embodiment of the present invention, the activator has a structure represented by the following formula: 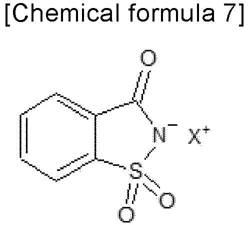 wherein
X is an organic base, forms a salt complex with saccharin, and is preferably N-methylimidazole, pyridine or 3-methylpyridine,
or by the following formula: 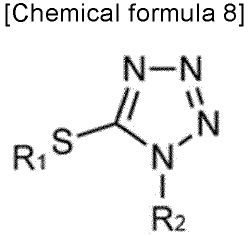
wherein
   R¹ and R² are each independently selected from the group consisting of H, linear or branched C₁₋₇ alkyl groups, and aromatic groups which may be optionally substituted.

Preferably, R¹ and R² are each independently H, a methyl group, an ethyl group, a propyl group, a butyl group, a pentyl group, a hexyl group, a phenyl group or a benzyl group, and more preferably, R¹ is H, CₙH₂ₙ₊₁ or a benzyl group, R² is H, CH₃, or C₆H₅, and n is 1, 2 or 3.

In one embodiment of the present invention, the activator is, for example, 5-mercapto-1-methyltetrazole (1-Me-MCT): 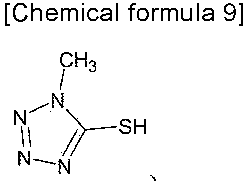
5-mercapto-1-phenyltetrazole (1-Ph-MCT): 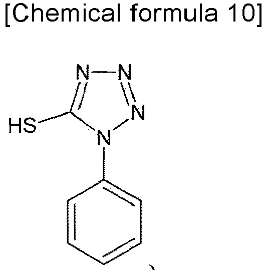
5-benzylthiotetrazole (BTT): 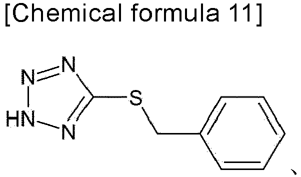
saccharin 1-methylimidazole (SMI): 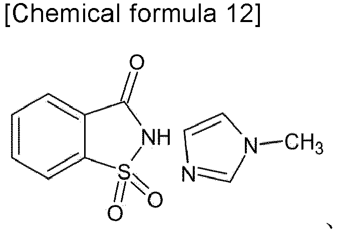
5-ethylthio-1H-tetrazole (ETT): 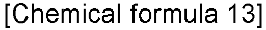 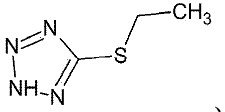
4,5-dicyanoimidazole (DCI): 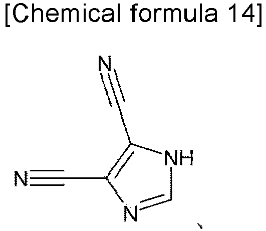
or benzimidazole trifluoromethanesulfonate (BIT):

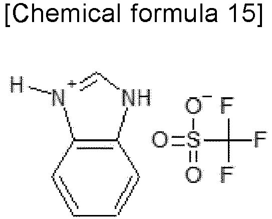

and it is preferably 1-Me-MCT, 1-Ph-MCT, SMI, ETT, or BTT, and more preferably 1-Me-MCT, 1-Ph-MCT, or SMI.

In one embodiment of the present invention, the HOMO energy (a.u.) of the activator of the present invention in acetonitrile is -0.22024 to -0.16858. For example, the HOMO energy (a.u.) of the activator of the present invention in acetonitrile is -0.21995 to -0.16858, -0.21407 to -0.16858, -0.19928 to - 0.16858, -0.18904 to -0.16858, or -0.18741 to -0.16858, and preferably - 0.21407 to -0.16858 (a.u.), and more preferably -0.18904 to -0.16858 (a.u.).

In one embodiment of the present invention, the orbital coefficient of the activator of the present invention in acetonitrile is 0.31531 to 0.59405. For example, the orbital coefficient of the activator of the present invention in acetonitrile is 0.31531 to 0.59405, 0.39931 to 0.59405, 0.51802 to 0.59405, or 0.53787 to 0.59405, and preferably 0.39931 to 0.59405.

In one embodiment of the present invention, the HOMO energy (a.u.) of the activator in acetonitrile is -0.22024 to -0.16858, and the orbital coefficient of said activator in acetonitrile is 0.31531 to 0.59405.

In one embodiment of the present invention, the HOMO energy (a.u.) of the activator in acetonitrile is -0.21407 to -0.16858, and the orbital coefficient of said activator in acetonitrile is 0.31531 to 0.59405.

The HOMO energy and orbital coefficient may be obtained by a quantum chemistry calculation program, for example, by an optimization calculation using Becke-style 3-parameter density functional theory (B3LYP) in the quantum chemistry calculation program Gaussian16 manufactured by Gaussian.

In one embodiment of the present invention, the pKa of the activator of the present invention in water at 25°C is 3.65 to 7.0; for example, 3.86 to 7.0, 4.1 to 7.0, 4.3 to 7.0, 4.5 to 7.0, 5.0 to 7.0, 5.5 to 7.0, 6.0 to 7.0, or 6.5 to 7.0.

In one embodiment of the present invention, in at least one coupling step in the method for producing an oligonucleotide, the nucleoside phosphoramidite is (a) a nucleoside phosphoramidite having two or more nucleoside moieties, or (b) a nucleoside phosphoramidite having one or more nucleoside moieties and a linker moiety.

In one embodiment of the present invention, in at least one coupling step in the method for producing an oligonucleotide, the nucleoside phosphoramidite is (a) a nucleoside phosphoramidite having two or three nucleoside moieties, or (b) a nucleoside phosphoramidite having one or more nucleoside moieties and a linker moiety.

A nucleoside phosphoramidite having two or three nucleoside moieties is presumed to have higher percentage of reaction, because of its smaller molecular size leading to higher motility and faster reaction rate, compared to a nucleoside phosphoramidite having four or more nucleoside moieties.

In one embodiment of the present invention, in at least the last one of the coupling steps performed two or more times in the method for producing an oligonucleotide, the nucleoside phosphoramidite is (a) a nucleoside phosphoramidite having two or more nucleoside moieties, or (b) a nucleoside phosphoramidite having one or more nucleoside moieties and a linker moiety.

In one embodiment of the present invention, in at least the last one of the coupling steps performed two or more times in the method for producing an oligonucleotide, the nucleoside phosphoramidite is a nucleoside phosphoramidite having three nucleoside moieties.

In one embodiment of the present invention, in at least one of the coupling steps performed two or more times in the method for producing an oligonucleotide, the nucleoside phosphoramidite is a nucleoside phosphoramidite having one nucleoside moiety.

In one embodiment of the present invention, only in the last one of the coupling steps performed two or more times in the method for producing an oligonucleotide, the nucleoside phosphoramidite is (a) a nucleoside phosphoramidite having three nucleoside moieties, or (b) a nucleoside phosphoramidite having one or more nucleoside moieties and a linker moiety, and in other coupling steps, the nucleoside phosphoramidite is a nucleoside phosphoramidite having one nucleoside moiety.

In the present invention, a nucleoside phosphoramidite having two or more nucleoside moieties can be prepared, for example, as described in WO 2019/212061.

In one embodiment of the present invention, the nucleoside phosphoramidite having two or more nucleoside moieties is a nucleoside phosphoramidite represented by the following formula (I): 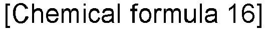 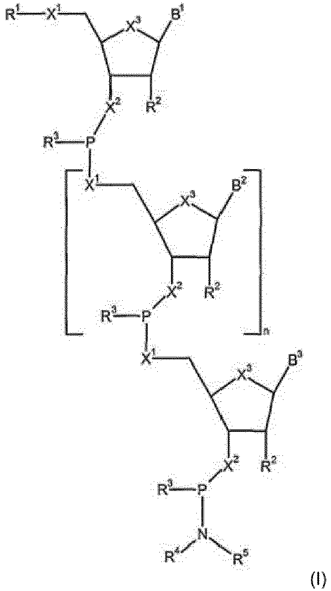 or a stereoisomer thereof.

In formula (I), X¹ is each independently -O- or -S-.

In formula (I), X² is each independently -O- or -S-.

In formula (I), X³ is each independently -O-, -S-, -CH₂- or -(CH₂)₂-.

In formula (I), R¹ is a protecting group, preferably an acid-labile protecting group, or a trialkylsilyl group such as t-butyldimethylsilyl or triisopropylsilyl. An acid-labile protecting group is a protecting group that can be removed by contacting the group with a protic acid or a Lewis acid. Acid-labile protecting groups are known to those skilled in the art. Examples of acid-labile protecting groups include a substituted or unsubstituted trityl group, a substituted or unsubstituted tetrahydropyranyl group, a substituted or unsubstituted tetrahydrofuranyl group, or a pixyl group, etc. Trityl groups are usually substituted with an electron donating group such as alkoxy groups. In a more preferred embodiment, R¹ is substituted or unsubstituted trityl, 9-phenylxanthenyl (hereinafter "pixyl") or tetrahydropyranyl (hereinafter "THP"). In an even more preferred embodiment, R¹ is unsubstituted trityl, monoalkoxytrityl, dialkoxytrityl, trialkoxytrityl, THP or pixyl. Most preferably R¹ is 4,4'-dimethoxytrityl.

In formula (I), R² is each independently H, NHR⁶, halogen, CN, CF₃, or any one of hydroxyl groups protected by an acyl-based protecting group, an ether-based protecting group or a silyl-based protecting group. Halogen is, for example, F, Cl, Br, and I. Examples of acyl-based protecting groups include acetyl, benzoyl, pivaloyl, etc. Examples of ether-based protecting groups include benzyl, p-methoxybenzyl (PMB), allyl, etc. Examples of silyl-based protecting groups include t-butyldimethylsilyl (TBS), t-butyldiphenylsilyl (TBDPS), t-triisopropylsilyl (TIPS), triethylsilyl (TES), trimethylsilyl (TMS), etc. It is preferably -H.

In formula (I), R³ is each independently -OCH₂CH₂CN, -SCH₂CH₂CN, a substituted or unsubstituted aliphatic group, -OR⁷ or -SR⁷, and it is preferably - OCH₂CH₂CN. Examples of substituted or unsubstituted aliphatic groups include, but are not limited to, 4-cyanobut-2-enylthio, 4-cyanobut-2-enyloxy, allylthio, allyloxy, crotylthio or crotyloxy, etc.

In formula (I), R⁴ and R⁵ are each independently a substituted or unsubstituted aliphatic group, a substituted or unsubstituted aromatic group, a substituted or unsubstituted aralkyl group; or R⁴ and R⁵ taken together with nitrogen to which they are bound form a heterocycloalkyl group or a heteroaromatic group. Examples of substituted or unsubstituted aliphatic groups include, but are not limited to, methyl, ethyl, isopropyl, etc., and preferably isopropyl. Examples of substituted or unsubstituted aromatic groups include, but are not limited to, phenyl, benzyl, naphthyl, 2-pyrenylmethyl, etc., and preferably phenyl, benzyl. Examples of substituted or unsubstituted aralkyl groups include, but are not limited to, 2-fluorophenylmethoxypiperidine-4-yl, etc. Examples of heterocycloalkyl groups include, but are not limited to, pyrrolidino, morpholino, etc., and preferably morpholino.

In formula (I), R⁶ is each independently -H, a substituted or unsubstituted aliphatic group, a substituted or unsubstituted aromatic group, a substituted or unsubstituted aralkyl group, or any one of protecting groups including an acyl group. Examples of substituted or unsubstituted aliphatic groups include, but are not limited to, methyl, ethyl, allyl, 1-pentenyl, 2-methoxyethyl, etc., and preferably methyl, allyl, 2-methoxyethyl. Examples of substituted or unsubstituted aromatic groups include, but are not limited to, phenyl, benzyl, naphthyl, 2-pyrenylmethyl, etc., and preferably phenyl, benzyl. Examples of substituted or unsubstituted aralkyl groups include, but are not limited to, 2-fluorophenylmethoxypiperidine-4-yl, etc. Protecting groups are, for example, t-butyldimethylsilyl, trifluoroacetyl, tert-butoxycarbonyl, benzyloxycarbonyl, phthaloyl, p-toluenesulfonyl.

In formula (I), R⁷ is a substituted or unsubstituted aliphatic group, a substituted or unsubstituted aromatic group, or a substituted or unsubstituted aralkyl group. Examples of substituted or unsubstituted aliphatic groups include, but are not limited to, THP, 4-methoxytetrahydropyranyl, etc. Examples of substituted or unsubstituted aromatic groups include, but are not limited to, o-chlorophenyl or p-chlorophenyl, etc. Examples of substituted or unsubstituted aralkyl groups include, but are not limited to, 2-fluorophenylmethoxypiperidine-4-yl, etc.

In formula (I), B¹, B² and B³ are each independently H or a protected or unprotected base. Examples of protected or unprotected bases include, but are not limited to, naturally occurring bases such as adenine, guanine, cytosine, thymine, and uracil; and modified bases such as 7-deazaguanine, 7-deaza-8-azaguanine, 5-propynylcytosine, 5-propynyluracil, 7-deazaadenine, 7-deaza-8-azaadenine, 7-deaza-6-oxopurine, 6-oxopurine, 3-deazaadenosine, 2-oxo-5-methylpyrimidine, 2-oxo-4-methylthio-5-methylpyrimidine, 2-thiocarbonyl-4-oxo-5-methylpyrimidine, 4-oxo-5-methylpyrimidine, 2-aminopurine, 5-fluorouracil, 2,6-diaminopurine, 8-aminopurine, 4-triazolo-5-methylthymine, and 4-triazolo-5-methyluracil.

In formula (I), n is 0 or a positive integer, preferably an integer of 0 or more and 4 or less, and more preferably 0 or 1.

In one embodiment of the present invention, the nucleoside phosphoramidite having one or more nucleoside moieties and a linker moiety is a nucleoside phosphoramidite with a linker bound at the 5'-position of the nucleoside via a phosphorus atom, for example, via a phosphite, a phosphate, a thiophosphate or a dithiophosphate.

In one embodiment of the present invention, the nucleoside phosphoramidite having one or more nucleoside moieties and a linker moiety may be prepared from, as a raw material, a phosphoramidite having the following linker moieties available from Glen Research, but they are not limited thereto:
PC Linker Phosphoramidite (3-(4,4'-Dimethoxytrityl)-1-(2-nitrophenyl)-propan-1-yl-[(2-cyanoethyl)-(N,N-diisopropyl)]-phosphoramidite)

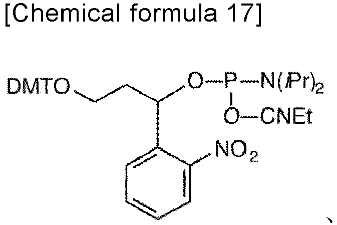

### 5'-Aminooxy-Modifier-11-CE Phosphoramidite (10-[N-dimethoxytritylaminooxyethyl)]-triethyleneglycol-1-[(2-cyanoethyl)-(N,N-diisopropyl)]-phosphoramidite)

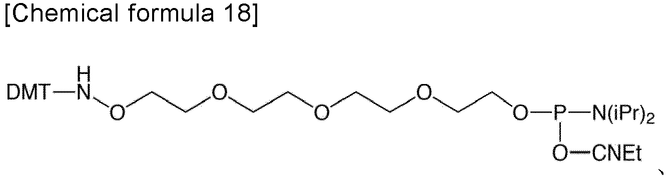

### α-Tocopherol-TEG Phosphoramidite (1-dimethoxytrityloxy-3-O-[(9-DL-α-tocopheryl)-triethyleneglycol-1-yl]-glyceryl-2-O-[(2-cyanoethyl)-(N,N,-diisopropyl)]-phosphoramidite)

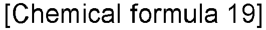

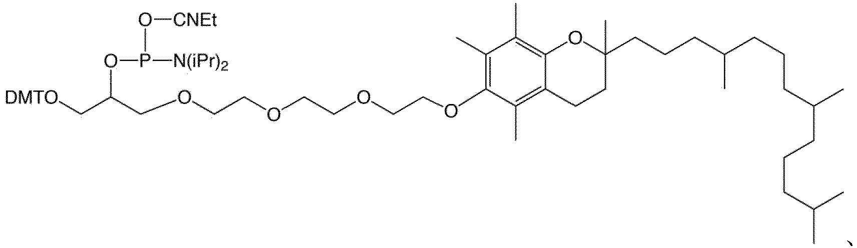

### 5'-DBCO-TEG Phosphoramidite (10-(6-oxo-6-(dibenzo[b,f]azacyclooct-4-yn-1-yl)-caproamido-N-ethyl)-O-triethyleneglycol-1-[(2-cyanoethyl)-(N,N-diisopropyl)]-phosphoramidite)

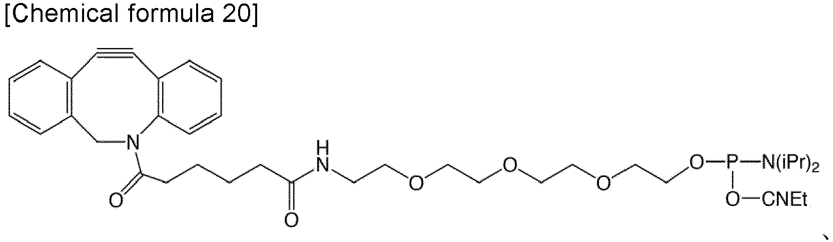

### 5'-Cholesteryl-TEG Phosphoramidite (10-0-[1-propyl-3-N-carbamoylcholesteryl]-triethyleneglycol-1-[(2-cyanoethyl)-(N,N-diisopropyl)]-phosphoramidite)

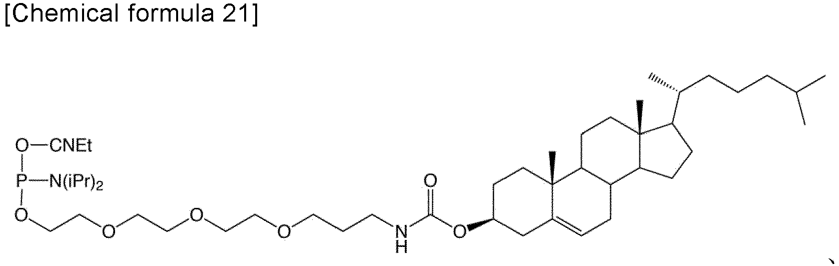

### DNP-TEG Phosphoramidite (1-Dimethoxytrityloxy-3-O-[N-(2,4-dinitrophenyl)-3-N-aminopropyl-(triethyleneglycol)]-glyceryl-2-O-(2-cyanoethyl)-(N,N-diisopropyl)-phosphoramidite)

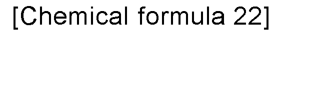

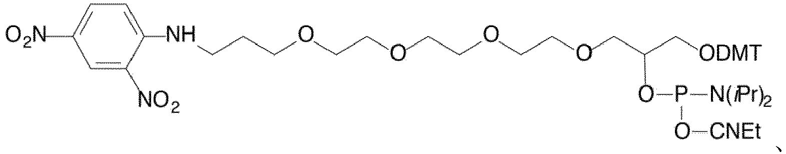

### Cholesteryl-TEG Phosphoramidite (1-Dimethoxytrityloxy-3-O-(N-cholesteryl-3-aminopropyl)-triethyleneglycol-glyceryl-2-O-(2-cyanoethyl)-(N,N,-diisopropyl)-phosphoramidite)

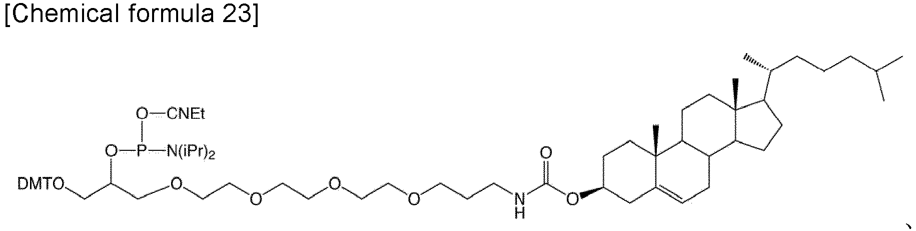

### 5'-Amino-Modifier TEG CE-Phosphoramidite (10-(O-trifluoroacetamido-N-ethyl)-triethyleneglycol-1-[(2-cyanoethyl)-(N,N-diisopropyl)]-phosphoramidite)

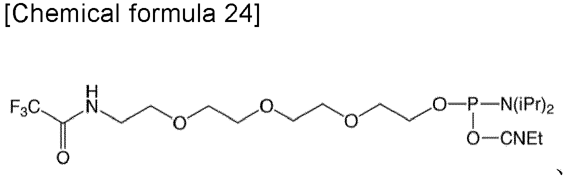

### Spacer Phosphoramidite 18 (18-O-Dimethoxytritylhexaethyleneglycol,1-[(2-cyanoethyl)-(N, N-diisopropyl)]-phosphoramidite)

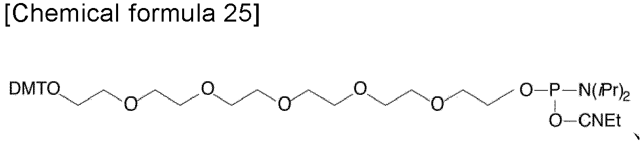

### Spacer Phosphoramidite 9 (9-O-Dimethoxytrityl-triethyleneglycol,1-[(2-cyanoethyl)-(N, N-diisopropyl)]-phosphoramidite)

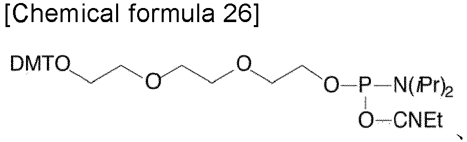

### PC Amino-Modifier Phosphoramidite ([(6-Trifluoroacetylamidocaproamidomethyl)-1-(2-nitrophenyl)-ethyl]-2-cyanoethyl-(N,N-diisopropyl)-phosphoramidite)

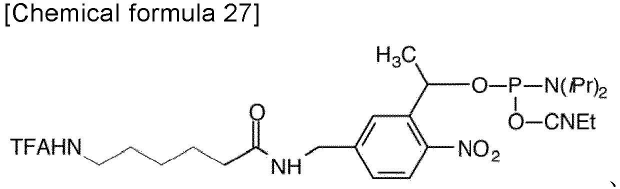

### Thiol-Modifier C6 S-S (1-O-Dimethoxytrityl-hexyl-disulfide,1'-[(2-cyanoethyl)-(N,N-diisopropyl)]-phosphoramidite)

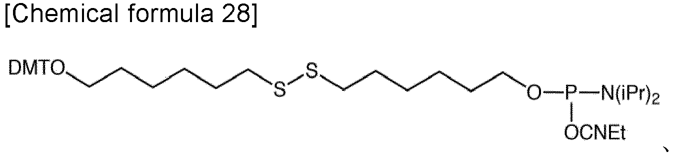

### 5'-Carboxy-Modifier C10 (10-Carboxy-decyl-(2-cyanoethyl)-(N,N-diisopropyl)-phosphoramidite, N-hydroxysuccinimide ester)

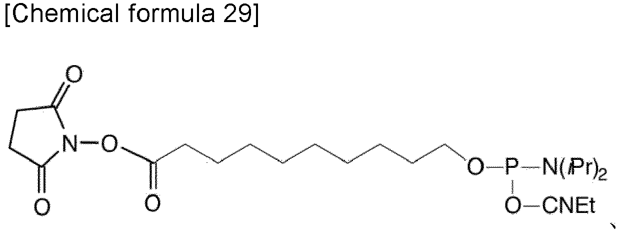

### 5'-Thiol-Modifier C6 (S-Trityl-6-mercaptohexyl-1-[(2-cyanoethyl)-(N,N-diisopropyl)]-phosphoramidite)

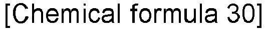

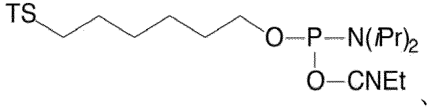

### Dithiol Serinol Phosphoramidite (3-Dimethoxytrityloxy-2-(3-((R)-α-lipoamido)propanamido)propyl-1-O-(2-cyanoethyl)-(N,N-diisopropyl)-phosphoramidite)

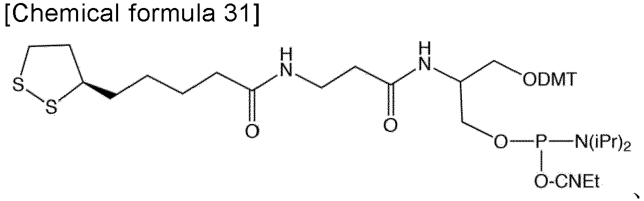

### 5'-Maleimide-Modifier Phosphoramidite (2-(1,7-Dimethyl-3,5-dioxo-10-oxa-4-azatricyclo[5.2.1.02,6]dec-8-en-4-yl)-ethyl-1-O-[(2-cyanoethyl)-(N,N-diisopropyl)]-phosphoramidite)

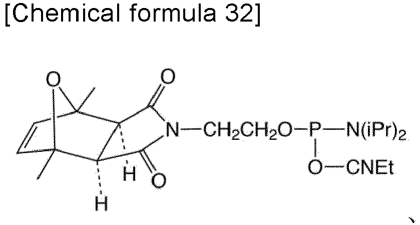

### PC Biotin Phosphoramidite (1-[2-Nitro-5-(6-(N-(4,4'-dimethoxytrityl))-biotinamidocaproamidomethyl)phenyl]-ethyl-[2-cyanoethyl-(N,N-diisopropyl)]-phosphoramidite)

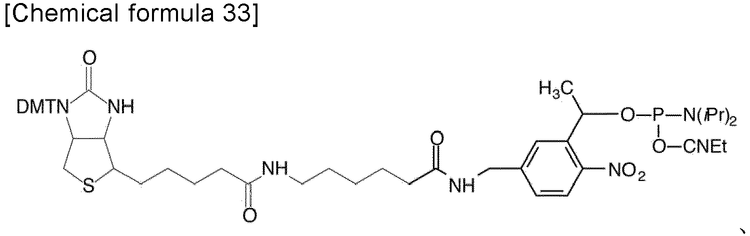

### Protected BiotinLC Serinol Phosphoramidite (3-Dimethoxytrityloxy-2-(3-((4-t-butylbenzoyl)-biotinyl-3-aminopropyl)-diethyleneglycolyl-propylamido-glycanoylamido)propyl-1-O-(2-cyanoethyl)-(N,N-diisopropyl)-phosphoramidite)

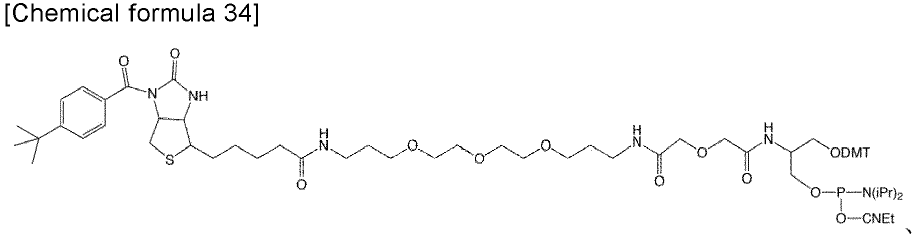

### 6-Fluorescein Serinol Phosphoramidite (3-Dimethoxytrityloxy-2-(3-(6-carboxy-(di-O-pivaloyl-fluorescein)propanamido)propyl)-1-O-(2-cyanoethyl)-(N,N-diisopropyl)-phosphoramidite)

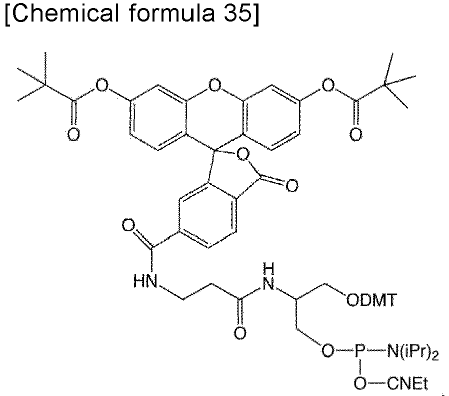

### Protected Biotin Serinol Phosphoramidite (3-Dimethoxytrityloxy-2-(3-((4-t-butylbenzoyl)-biotinyl)propanamido)propyl-1-O-(2-cyanoethyl)-(N,N-diisopropyl)-phosphoramidite)

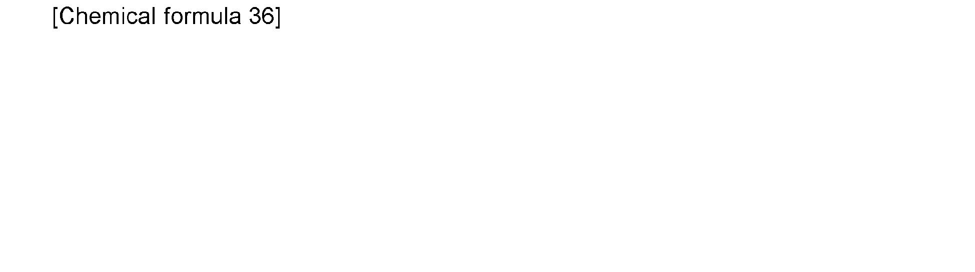

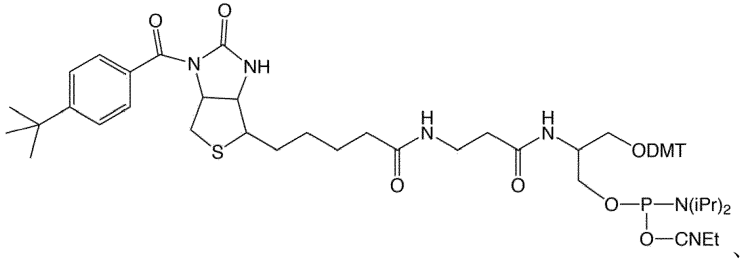

### 1-Ethynyl-dSpacer CE Phosphoramidite (5'-O-Dimethoxytrityl-1'-ethynyl-2'-deoxyribose-3'-[(2-cyanoethyl)-(N,N-diisopropyl)]-phosphoramidite)

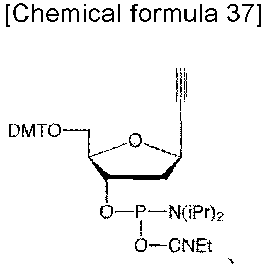

### Azobenzene Phosphoramidite (3-O-(Dimethoxytrityl)-2-N-(4-carboxyazobenzene)-D-threonin-1-yl-O-[(2-cyanoethyl)-(N,N-diisopropyl)]-phosphoramidite)

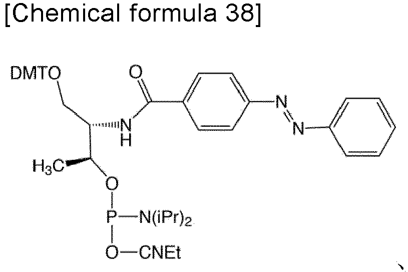

### 5'-I-dT-CE Phosphoramidite (5'-lodo-2'-deoxyThymidine,3'-[(2-cyanoethyl)-(N,N-diisopropyl)]-phosphoramidite)

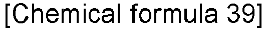

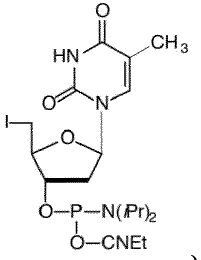

### Psoralen C6 Phosphoramidite (6-[4'-(Hydroxymethyl)-4,5',8-trimethylpsoralen]-hexyl-1-O-(2-cyanoethyl)-(N,N-diisopropyl)-phosphoramidite)

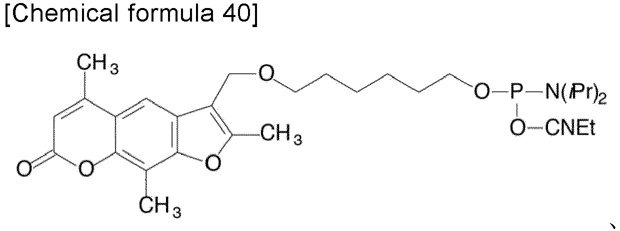

### Psoralen C2 Phosphoramidite (2-[4'-(Hydroxymethyl)-4,5',8-trimethylpsoralen]-ethyl-1-O-(2-cyanoethyl)-(N,N-diisopropyl)-phosphoramidite)

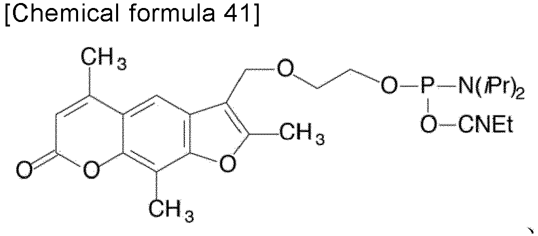

### 3-Cyanovinylcarbazole Phosphoramidite (CNVK) (5'-O-(4,4'-Dimethoxytrityl)-1'-(3-cyanovinylcarbazol-9-yl)-2'-deoxy-β-D-ribofuranosyl-3'-[(2-cyanoethyl)-(N,N-diisopropyl)]-phosphoramidite)

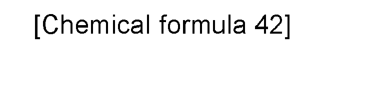

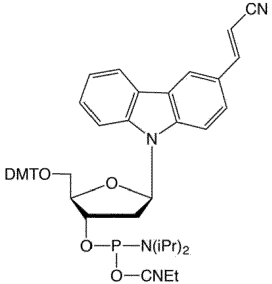

In one embodiment of the present invention, the nucleoside phosphoramidite having one or more nucleoside moieties and a linker moiety is a nucleoside phosphoramidite represented by the following formula (II): 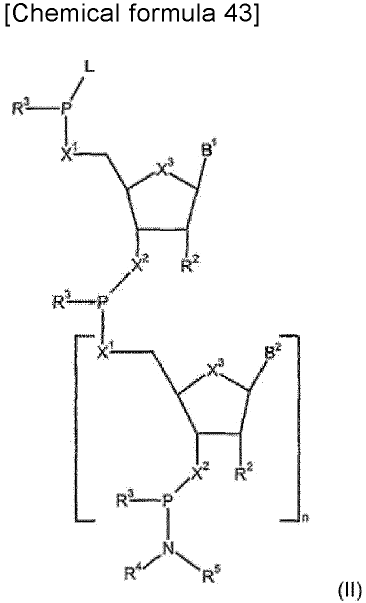 or a stereoisomer thereof.

In formula (II), X¹ is each independently -O- or -S-.

In formula (II), X² is each independently -O- or -S-.

In formula (II), X³ is each independently -O-, -S-, -CH₂- or -(CH₂)₂-.

In formula (II), L is a linker. Examples of the linker include, but are not limited to, the following and the like:

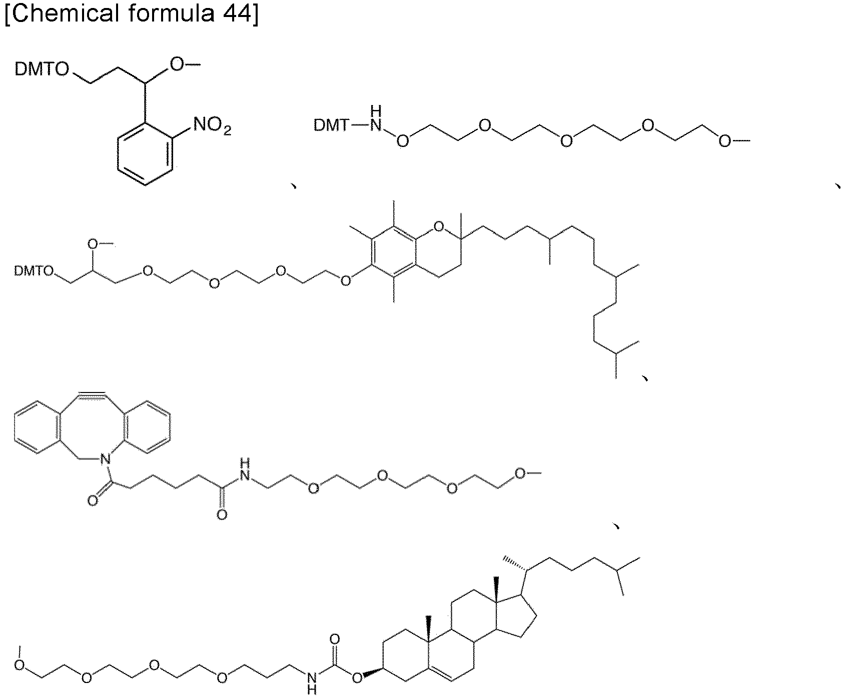

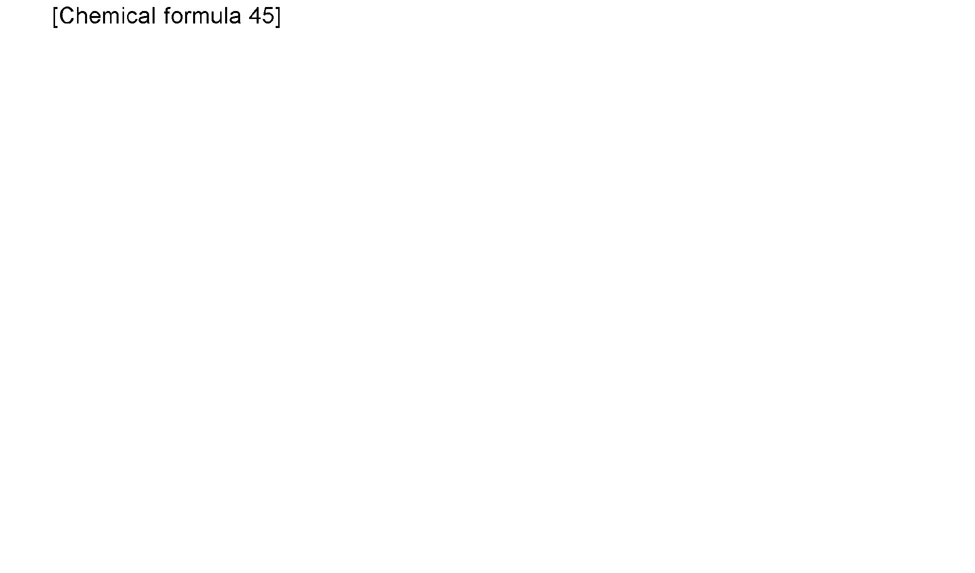

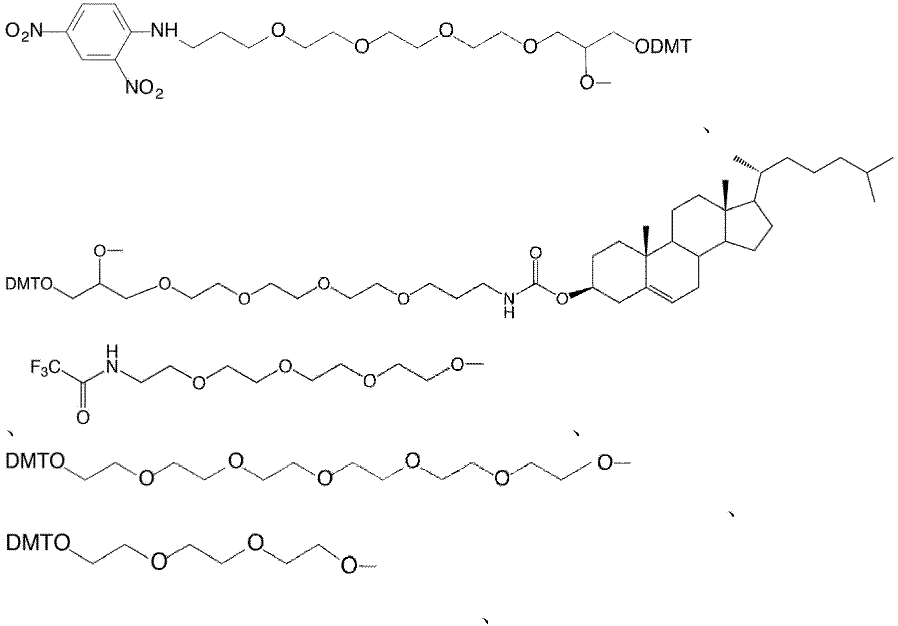

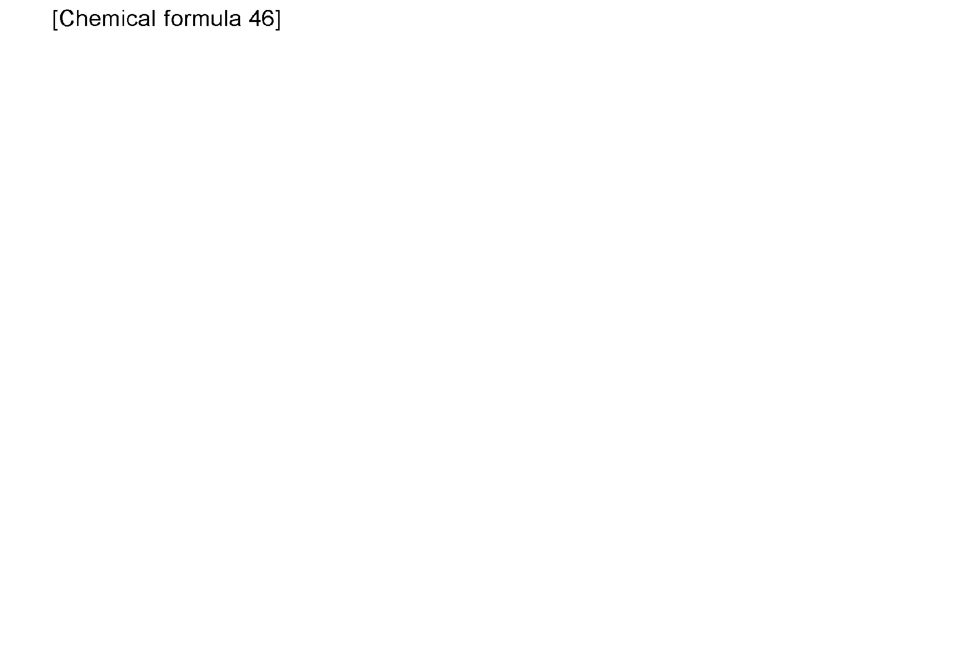

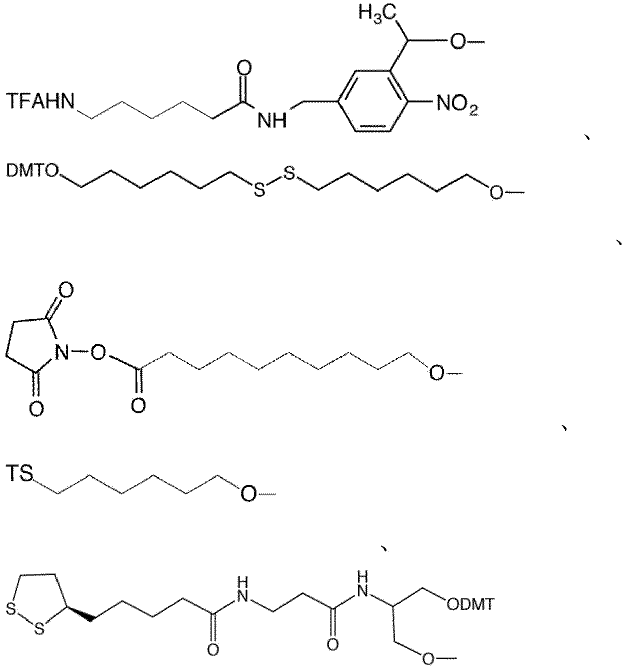

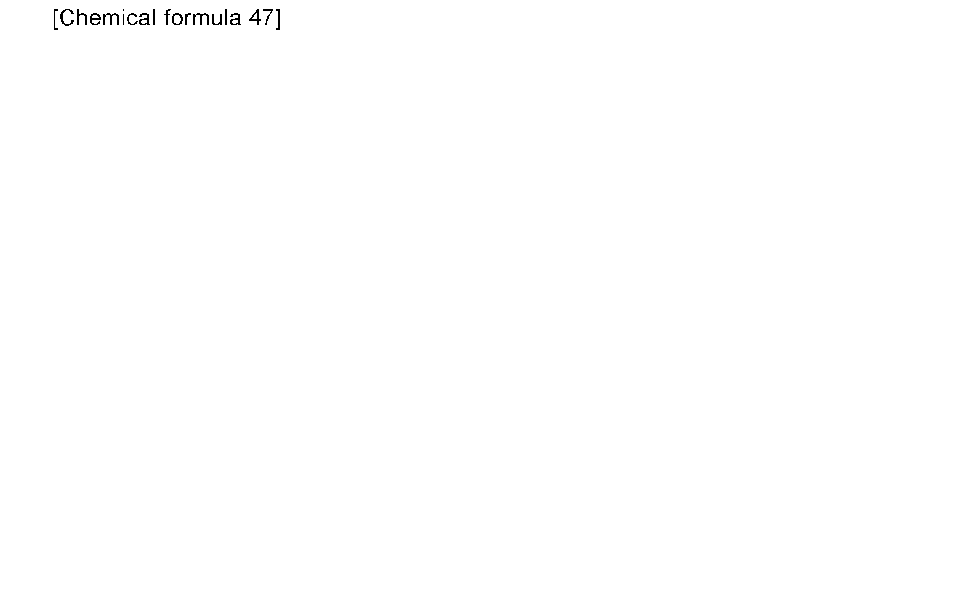

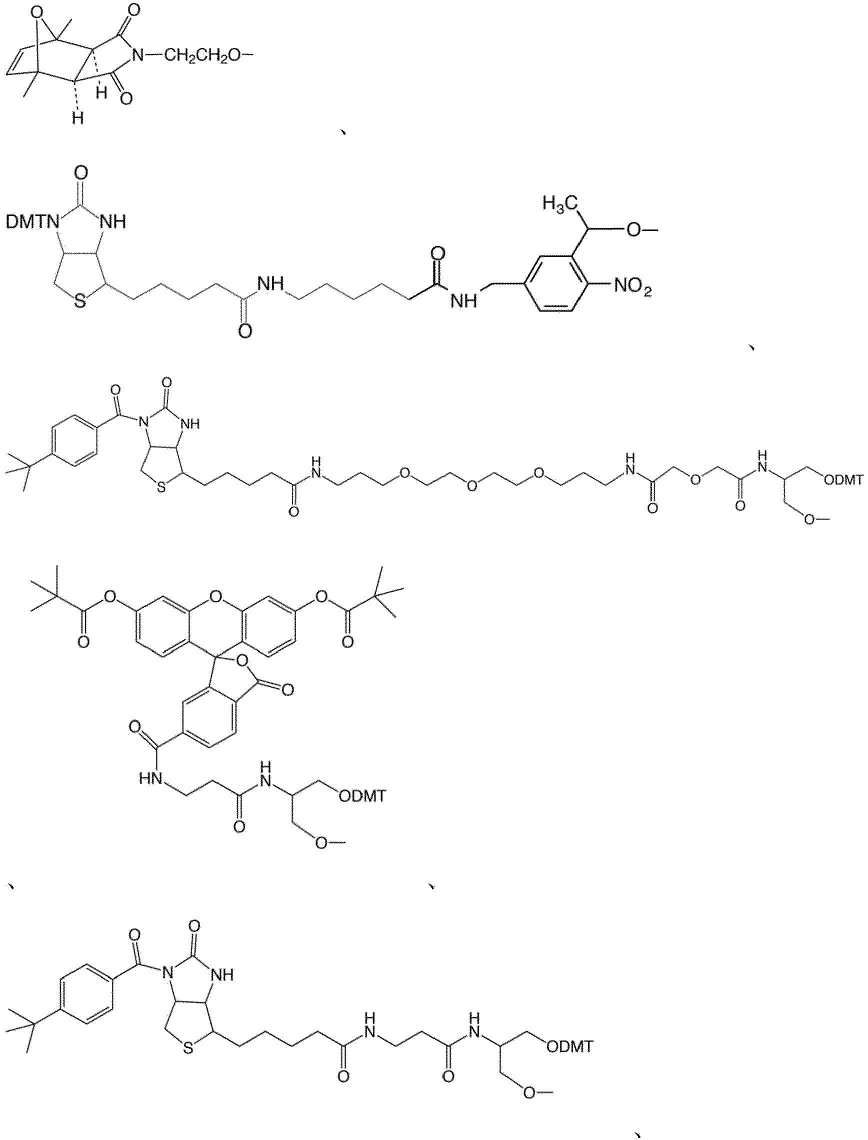

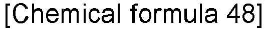

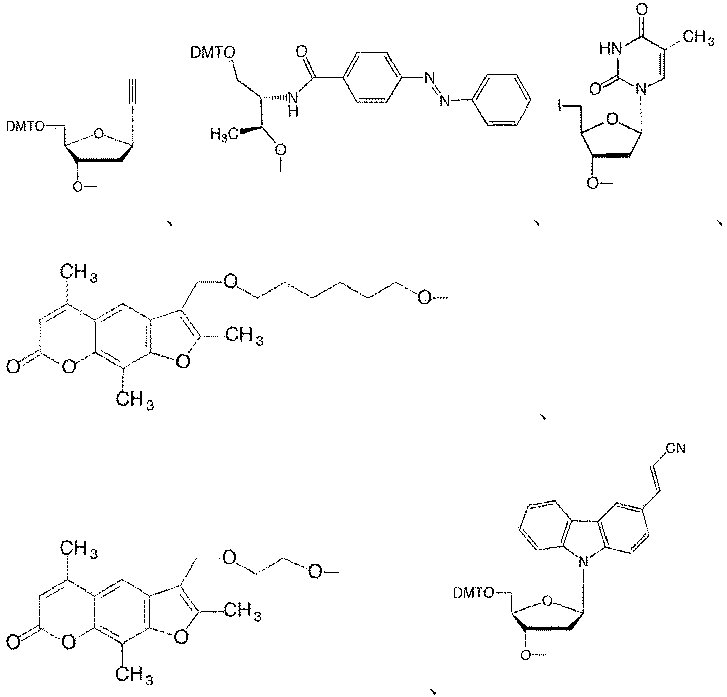

In formula (II), R² is each independently -H, -NHR⁶, halogen, -CN, - CF₃, or any one of hydroxyl groups protected by an acyl-based protecting group, an ether-based protecting group or a silyl-based protecting group. Halogen is, for example, F, Cl, Br, and I. Examples of acyl-based protecting groups include acetyl, benzoyl, pivaloyl, etc. Examples of ether-based protecting groups include benzyl, p-methoxybenzyl (PMB), allyl, etc. Examples of silyl-based protecting groups include t-butyldimethylsilyl (TBS), t-butyldiphenylsilyl (TBDPS), t-triisopropylsilyl (TIPS), triethylsilyl (TES), trimethylsilyl (TMS), etc. It is preferably -H.

In formula (II), R³ is each independently -OCH₂CH₂CN, -SCH₂CH₂CN, a substituted or unsubstituted aliphatic group, -OR⁷ or -SR⁷, and it is preferably -OCH₂CH₂CN. Examples of substituted or unsubstituted aliphatic groups include, but are not limited to, 4-cyanobut-2-enylthio, 4-cyanobut-2-enyloxy, allylthio, allyloxy, crotylthio or crotyloxy, etc.

In formula (II), R⁴ and R⁵ are each independently a substituted or unsubstituted aliphatic group, a substituted or unsubstituted aromatic group, a substituted or unsubstituted aralkyl group; or R⁴ and R⁵ taken together with nitrogen to which they are bound form a heterocycloalkyl group or a heteroaromatic group. Examples of substituted or unsubstituted aliphatic groups include, but are not limited to, methyl, ethyl, isopropyl, etc., and preferably isopropyl. Examples of substituted or unsubstituted aromatic groups include, but are not limited to, phenyl, benzyl, toluyl, anilyl, etc., and preferably phenyl, benzyl. Examples of heterocycloalkyl groups include, but are not limited to, pyrrolidino, morpholino, etc.

In formula (II), R⁶ is each independently -H, a substituted or unsubstituted aliphatic group, a substituted or unsubstituted aromatic group, a substituted or unsubstituted aralkyl group, or any one of protecting groups including an acyl group. Examples of substituted or unsubstituted aliphatic groups include, but are not limited to, methyl, ethyl, allyl, 1-pentenyl, 2-methoxyethyl, etc., and preferably methyl, allyl, 2-methoxyethyl. Examples of substituted or unsubstituted aromatic groups include, but are not limited to, phenyl, benzyl, naphthyl, 2-pyrenylmethyl, etc., and preferably phenyl, benzyl. The protecting group is, for example, t-butyldimethylsilyl.

In formula (II), R⁷ is a substituted or unsubstituted aliphatic group, a substituted or unsubstituted aromatic group, or a substituted or unsubstituted aralkyl group. Examples of substituted or unsubstituted aliphatic groups include, but are not limited to, THP, 4-methoxytetrahydropyranyl, etc. Examples of substituted or unsubstituted aromatic groups include, but are not limited to, o-chlorophenyl or p-chlorophenyl, etc. Examples of substituted or unsubstituted aralkyl groups include, but are not limited to, 2-fluorophenylmethoxypiperidine-4-yl, etc.

In formula (II), B¹, and B² are each independently H or a protected or unprotected base. Examples of protected or unprotected bases include, but are not limited to, naturally occurring bases such as adenine, guanine, cytosine, thymine, and uracil; and modified bases such as 7-deazaguanine, 7-deaza-8-azaguanine, 5-propynylcytosine, 5-propynyluracil, 7-deazaadenine, 7-deaza-8-azaadenine, 7-deaza-6-oxopurine, 6-oxopurine, 3-deazaadenosine, 2-oxo-5-methylpyrimidine, 2-oxo-4-methylthio-5-methylpyrimidine, 2-thiocarbonyl-4-oxo-5-methylpyrimidine, 4-oxo-5-methylpyrimidine, 2-aminopurine, 5-fluorouracil, 2,6-diaminopurine, 8-aminopurine, 4-triazolo-5-methylthymine, and 4-triazolo-5-methyluracil. In a preferred embodiment, at least one of B¹ and B² is adenine.

In formula (II), n is 0 or a positive integer, preferably an integer of 0 or more and 4 or less, and more preferably 0 or 1.

### [Examples]

The present invention will be described in more detail with reference to the following examples, which show specific examples of the present invention; the present invention is not limited thereto.

### Example 1: Comparison between sequential method and segment method

### (1) Synthesis of oligonucleotides

A 17mer (5' CCG ATT AAG CGAAGC TT 3') DNA oligonucleotide was synthesized by filling a reaction column with dT NittoPhase^{®} HL dT300 (Nitto Denko Corporation) in an amount corresponding to 1573 µmοl, and using an nucleic acid synthesizer AKTA oligopilot plus100 (Cytiva, the former GE Healthcare Japan). Then, a solid phase carrier attached with 17mer DNA was filled in the reaction column in an amount corresponding to 205 µmοl, and the synthesis was carried out with the following three conditions, using the nucleic acid synthesizer AKTA oligopilot plus100 with the use of 5-ethylthio-1H-tetrazole (ETT) as an activator: a sequential method wherein dC, dT, dA are condensed sequentially; a segment method 1 wherein dATC with an amidite equivalent of 1.8 equivalents was condensed with condensation time of 5 minutes; and a segment method 2 wherein dATC with an amidite equivalent of 3.0 equivalents was condensed with condensation time of 10 minutes. As other synthetic reagents, commonly used deprotecting reagent, capping reagent and oxidizing solution were used. dATC was made as described in WO 2019/212061.

The solid phase carrier to which the DNA oligonucleotide was bound was immersed in 28% ammonia water, and said DNA oligonucleotide was cut out from the solid phase carrier. A part of this solution was diluted with water to prepare a DNA oligonucleotide sample solution. The remaining solution was used as a crude solution.

### (2) Purification of oligonucleotides

The obtained crude solutions were purified by a purification equipment AKTA pure 25 (Cytiva, the former GE Healthcare Japan) (purification conditions: column: 25 cm x 10 mm, CV = 19.6 mL GE source 15Q resin, Buffer: NaOH, NaCl, adjusted with ultrapure water).

### (3) Analysis of DNA oligonucleotides

The DNA oligonucleotide sample solutions before and after purification were measured by high performance liquid chromatography (HPLC) (measurement conditions: column: Waters XBridge OST C18, 2.5 µm, 50 x 4.6 mm, UV detection: 260 nm, Buffer A: HFIP/TEA in water, Buffer B: methanol).

### (4) Results

Table 1 shows the analysis results of the DNA oligonucleotide sample solutions before purification.

**[Table 1]**

| | Sequential method | Segment method 1 | Segment method 2 |
|---|---|---|---|
| Sample ID | mi09122018-1 | mi09122018-2 | mi09122018-3 |
| Purity (%) | 73.8 | 72.2 | 75.6 |
| Absorbance (A260) | 439 | 409 | 436 |
| Total OD | 21925 | 20450 | 21800 |
| FLP (Purity x Total OD) | 16181 | 14765 | 16481 |
| Impurity I (%) | 2.9 | 0.6 | 0.7 |
| Impurity II (%) | 2.8 | 0.4 | 1.8 |
| Impurity III (%) | 1.5 | 7.9 | 2.8 |

Here, Impurity I refers to an impurity that appears near (N-1)mer in HPLC measurement, Impurity II refers to an impurity that appears near (N-2)mer in HPLC measurement, and Impurity III refers to an impurity that appears near (N-3)mer in HPLC measurement. The same applies below.

When synthesized by the sequential method, the contents of Impurity I and Impurity II were 2.9% and 2.8%, respectively, whereas when synthesized by the segment method 1, the contents were 0.6% and 0.4%, respectively, and when synthesized by the segment method 2, the contents were 0.7% and 1.8%, respectively. When synthesized by the segment methods, the contents of Impurity I and Impurity II could be significantly reduced as compared with the case of synthesizing by the sequential method.

Table 2 shows the analysis results of the DNA oligonucleotide sample solutions after purification.

**[Table 2]**

| | Sequential method | Segment method 1 | Segment method 2 |
|---|---|---|---|
| Purity (%) | 90.46 | 92.34 | 92.53 |
| Absorbance (A260) | 0.451 | 0.439 | 0.439 |
| Total OD | 10283 | 11590 | 11590 |
| FLP (Purity x Total OD) | 9302 | 10702 | 10724 |
| Impurity I (%) | 2.47 | 0.41 | 0.42 |
| Impurity II (%) | 0.47 | 0.62 | 1.01 |
| Impurity III (%) | 0.26 | 0.25 | 0.29 |

The purity of the target oligonucleotide was 90.46% in the sequential method, 92.34% in the segment method 1 and 92.53% in the segment method 2; when synthesized by the segment methods, the target oligonucleotide could be obtained with high purity as compared with the case of synthesizing by the sequential method. In addition, the FLP (full-length product, purity x total OD) was 9302 in the sequential method, whereas it was 10702 in the segment method 1 and 10724 in the segment method 2; and when synthesized by the segment methods, the target oligonucleotide could be obtained in a high yield as compared with the case of synthesizing by the sequential method.

### Example 2: Comparison of percentage of reaction

### (1) Synthesis of DNA oligonucleotides

NittoPhase^{®} HL UnyLinker 350 (Nitto Denko Corporation) was filled in a reaction column in an amount equivalent to 2935 µmοl, and a 17mer (5' CCG ATT AAG CGAAGC TT 3') DNA oligonucleotide was synthesized using the nucleic acid synthesizer AKTA oligopilot plus100. Then, a solid phase carrier attached with the 17mer DNA was filled in the reaction column in an amount corresponding to 90 µmοl, and dGCC with an amidite equivalent amount of 1.8 equivalents was condensed with condensation time of 5 minutes using the nucleic acid synthesizer AKTA oligopilot plus10 and the following activators: 5-mercapto-1-methyltetrazole (1-Me-MCT), 5-mercapto-1-phenyltetrazole (1-Ph-MCT), 5-benzylthiotetrazole (BTT), saccharin 1-methylimidazole (SMI), 5-ethylthio-1H-tetrazole (ETT), 4,5-dicyanoimidazole (DCI), 5-[3,5-bis(trifluoromethyl)phenyl]-1H-tetrazole (Activator42), or benzimidazole trifluoromethanesulfonate (BIT). All activators were dissolved in acetonitrile to adjust to 0.25 M. Other synthetic reagents used include: 3% DCA in toluene as a deprotecting agent; pyridine, iodine in water as an oxidizing agent; pyridine, N-methylimidazole, acetic anhydride or isobutyric anhydride in acetonitrile as a capping agent; and TBA in acetonitrile as an amine wash reaction solution. The dGCC was prepared as described in WO 2019/212061.

The solid phase carrier to which the DNA oligonucleotide was bound was immersed in aqueous ammonia, and said DNA oligonucleotide was cut out from the solid phase carrier.

Here, Activator42 has the following structure:

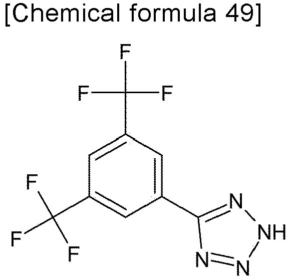

### (2) Synthesis of RNA oligonucleotides

NittoPhase^{®} HL rU250 (Kinovate Life Science) was filled in a reaction column in an amount equivalent to 327 µmοl, and a 17mer (5' CCG AUU AAG CGAAGC UU 3') RNA oligonucleotide was synthesized using the nucleic acid synthesizer AKTA oligopilot plus100. Then, a solid phase carrier attached with the 17mer RNA was filled in the reaction column in an amount corresponding to 85 µmοl, and rAUC with an amidite equivalent amount of 2.0 equivalents was condensed with condensation time of 15 minutes using the nucleic acid synthesizer AKTA oligopilot plus10 and the following activators: 5-mercapto-1-methyltetrazole (1-Me-MCT), saccharin 1-methylimidazole (SMI), or 5-[3,5-bis(trifluoromethyl)phenyl]-1H-tetrazole (Activator42). All activators were dissolved in acetonitrile to adjust to 0.25 M. Other synthetic reagents used include: 3% DCA in toluene as a deprotecting agent; pyridine, iodine in water as an oxidizing agent; pyridine, N-methylimidazole, acetic anhydride in acetonitrile as a capping agent; and TBA in acetonitrile as an amine wash reaction solution. The rAUC was prepared as described in WO 2019/212061.

The solid phase carrier to which the RNA oligonucleotide was bound was immersed in an AMA reagent (28-30% aqueous ammonia : aqueous methylamine solution = 1 : 1), filtered through a filter, and washed with DMSO. Then, by slowly dropping TEA. 3HF in an ice bath and by shaking, said RNA oligonucleotide was cut out from the solid phase carrier.

### (3) Analysis of percentage of reaction

Each oligonucleotide sample solution after the cutting out was measured by high performance liquid chromatography (HPLC) (measurement conditions: column: Waters XBridge OST C18, 2.5 µm, 50 x 4.6 mm, UV detection: 260 nm, Buffer A: HFIP/TEA in water, Buffer B: methanol).

In the DNA synthesis, the sum of the peak areas detected within 1.1 minutes after observation of the peak of Impurity III in the HPLC measurement result was set to 100%, from which the peak area (%) of Impurity III was subtracted, to obtain the percentage of reaction. In the RNA synthesis, the sum of the peak areas detected within 1.4 minutes after observation of the peak of Impurity III in the HPLC measurement result was set to 100%, from which the peak area (%) of Impurity III was subtracted, to obtain the percentage of reaction.

### (4) Results

Table 3 shows results in the DNA synthesis, and Table 4 shows results in the RNA synthesis.

The HOMO energy and orbital coefficient of the activators used in this experiment are also shown in Tables 3 and 4. The HOMO energy and orbital coefficient were obtained by optimization calculation using Becke-style 3-parameter density functional theory (B3LYP) in the quantum chemistry calculation program Gaussian16 manufactured by Gaussian. Specifically, the structure of the active species (BIT is a neutral species, the others are anionic species) when the activator makes a nucleophilic attack on amidite, that is, the initial structure of the activator, is determined as follows: the initial structure of the molecule before calculation was generated by using Web-based calculation support program WebMO; and 6-31G (d) was input in the basis function, -1 for anion and 0 for neutral species were input in the charge, single term was input in the multiplicity, and acetonitrile was input in the solvent; then by executing structural optimization and orbital calculation in this order, HOMO energy and orbital coefficient were obtained. A simple structural modification of the initial structure to avoid collisions between atoms in the molecular model was appropriately performed by executing "Mechanics Optimize" of the Cleanup function in WebMO.

### [Table 3]

**Table 3 Percentage of reaction for dGCC amidite**

| Activator | Percentage of reaction | Percentage of reaction (%) | Impurity III | Nucleophilic parameter (in acetonitrile) | | | |
|---|---|---|---|---|---|---|---|
| | Ranking | | Peak area (%) | Rank ing | HOMO energy (a.u.) | Rank ing | Orbital coefficient |
| 1-Me-MCT | 1 | 89.21 | 10.791 | 2 | -0.18741 | 2 | 0.56513 |
| 1-Ph-MCT | 2 | 88.7 | 11.303 | 3 | -0.18904 | 3 | 0.53787 |
| BTT | 3 | 87.79 | 12.207 | 7 | -0.22024 | 1 | 0.59405 |
| SMI | 4 | 85.42 | 14.583 | 1 | -0.16858 | 5 | 0.39931 |
| ETT | 5 | 85.26 | 14.744 | 4 | -0.19928 | 4 | 0.51802 |
| DCI | 6 | 80.25 | 19.75 | 5 | -0.21407 | 6 | 0.31531 |
| Activator42 | 7 | 78.52 | 21.485 | 6 | -0.21995 | 7 | 0.29531 |
| BIT | 8 | 73.21 | 26.792 | 8 | -0.22833 | 8 | 0.28513 |

### [Table 4]

**Table 4 Percentage of reaction for rAUC amidite**

| Activator | Percentage of reaction | Percentage of reaction (%) | Impurity III |
|---|---|---|---|
| | Ranking | | Peak area (%) |
| 1-Me-MCT | 1 | 82.768 | 17.232 |
| SMI | 2 | 75.998 | 24.002 |
| Activator42 | 3 | 74.946 | 25.054 |

From Tables 3 and 4, it can be seen that the activators showing a higher percentage of reaction than Activator42 had a higher HOMO energy level and/or a larger orbital coefficient. It is considered that a high HOMO energy level of the activator reduces the energy difference between the HOMO energy level of the activator and the LUMO energy level of the segment-type amidite, thereby facilitating the reaction. In addition, it is considered that a large orbital coefficient of the activator increases the overlap of the orbitals between nitrogen atoms of the activator and phosphorus atoms of the segment-type amidite, thereby facilitating the reaction.

### Example 3. Comparison of degradation of segment-type amidites

### (1) Synthesis of oligonucleotides using dGCC as a segment-type amidite

The solid phase carrier attached with the 17mer DNA obtained in Example 2 (1) was filled in a reaction column in an amount corresponding to 90 µmol, and dGCC with an amidite equivalent amount of 1.8 equivalents was condensed with condensation time of 10 minutes using the nucleic acid synthesizer AKTA oligopilot plus10 and the following activators: 0.6M 5-mercapto-1-methyltetrazole (1-Me-MCT), 0.5M 5-mercapto-1-phenyltetrazole (1-Ph-MCT), 0.25M 5-benzylthiotetrazole (BTT), 0.25M saccharin 1-methylimidazole (SMI), 0.6M 5-ethylthio-1H-tetrazole (ETT) or 0.25M 5-[3,5-bis(trifluoromethyl)phenyl]-1H-tetrazole (Activator42). Other synthetic reagents used include: 3% DCA in toluene as a deprotecting agent; pyridine, iodine in water as an oxidizing agent; pyridine, N-methylimidazole, acetic anhydride in acetonitrile as a capping agent; and TBA in acetonitrile as an amine wash reaction solution. The dGCC was prepared as described in WO 2019/212061.

The solid phase carrier to which the DNA oligonucleotide was bound was immersed in aqueous ammonia at 55°C for 12 to 16 hours, and said DNA oligonucleotide was cut out from the solid phase carrier.

### (2) Analysis of DNA oligonucleotides

The cut-out DNA oligonucleotide sample solutions were measured by high performance liquid chromatography (HPLC) (measurement conditions: column: Waters XBridge OST C18, 2.5 µm, 50 x 4.6 mm, UV detection: 260 nm, Buffer A: 100-mM HFIP/7-mM TEA in water, pH 8.0, Buffer B: methanol, temperature: 60°C).

The sum of the peak areas detected within 1.1 minutes after observation of the peak of Impurity III in the HPLC measurement result was set to 100%, and the peak area (%) of Impurity II was calculated.

### (4) Results

The results are shown in Table 5. Table 5 also shows pKa values in water of the activators used in this experiment.

### [Table 5]

**Table 5 Percentage of Impurity II**

| Activator | pKa | Impurity II |
|---|---|---|
| | in H₂O | Peak area (%) |
| Activator42 | 3.4 | 9.089 |
| 1-Ph-MCT | 3.65 | 2.625 |
| 1-Me-MCT | 3.86 | 2.402 |
| BTT | 4.1 | 2.857 |
| ETT | 4.3 | 1.748 |
| SMI | 7 | 1.583 |

In the case of synthesis using Activator42, the peak area of Impurity II was 9.089%, and remarkable degradation of segment-type amidites was observed. In contrast, when other activators were used, the peak area of Impurity II was approximately 1 to 2%, and almost no degradation was observed. It is presumed that Impurity II is likely to be generated by degradation by acid of the phosphodiester bond that connects the oligonucleotide and the segment-type amidite.

## Claims

1. A method for producing an oligonucleotide, comprising performing one or more coupling steps of binding a nucleoside phosphoramidite to a 3' or 5' hydroxyl or thiol group of a nucleotide or nucleoside in the presence of an activator,
wherein in at least one coupling step, the nucleoside phosphoramidite is
(a) a nucleoside phosphoramidite having two or more nucleoside moieties, or
(b) a nucleoside phosphoramidite having one or more nucleoside moieties and a linker moiety, and
the activator has a structure represented by the following formula: 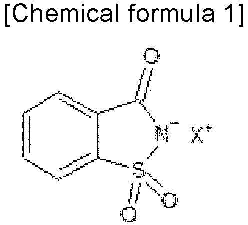
wherein X is an organic base,
or by the following formula: 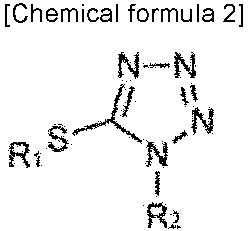
wherein R¹ and R² are each independently selected from the group consisting of H, linear or branched C₁₋₇ alkyl groups, and aromatic groups which may be optionally substituted.

2. The method according to claim 1, wherein
X is N-methylimidazole, pyridine or 3-methylpyridine,
R¹ is H, CₙH₂ₙ₊₁ or a benzyl group,
R² is H, CH₃, or C₆H₅, and
n is 1, 2 or 3.

3. The method according to claim 1 or 2, wherein the activator is 5-mercapto-1-methyltetrazole (1-Me-MCT), 5-mercapto-1-phenyltetrazole (1-Ph-MCT), saccharin 1-methylimidazole (SMI), or 5-ethylthio-1H-tetrazole (ETT).

4. The method according to any one of claims 1 to 3, wherein in at least the last one of the coupling steps performed two or more times,
the nucleoside phosphoramidite is
(a) a nucleoside phosphoramidite having two or more nucleoside moieties, or
(b) a nucleoside phosphoramidite having one or more nucleoside moieties and a linker moiety.

5. The method according any one of claims 1 to 4, wherein in at least the last one of the coupling steps performed two or more times,
the nucleoside phosphoramidite is a nucleoside phosphoramidite having three nucleoside moieties.

6. The method according to any one of claims 1 to 5, wherein in at least one of the coupling steps performed two or more times,
the nucleoside phosphoramidite is a nucleoside phosphoramidite having one nucleoside moiety.

7. The method according to any one of claims 1 to 6, wherein only in the last one of the coupling steps performed two or more times,
the nucleoside phosphoramidite is
(a) a nucleoside phosphoramidite having three nucleoside moieties, or
(b) a nucleoside phosphoramidite having one or more nucleoside moieties and a linker moiety, and
in other coupling steps,
the nucleoside phosphoramidite is a nucleoside phosphoramidite having one nucleoside moiety.

8. The method according to any one of claims 1 to 7, wherein the nucleoside phosphoramidite having two or more nucleoside moieties is a nucleoside phosphoramidite represented by the following formula (I): 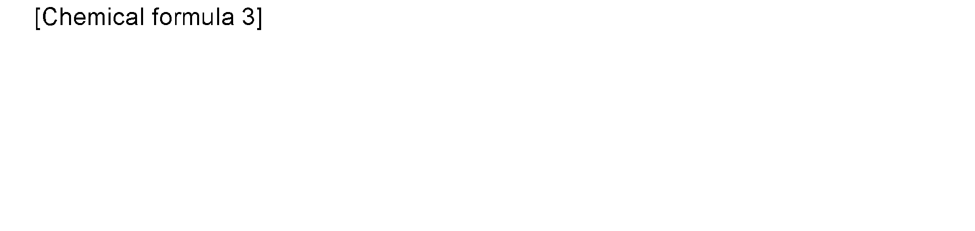 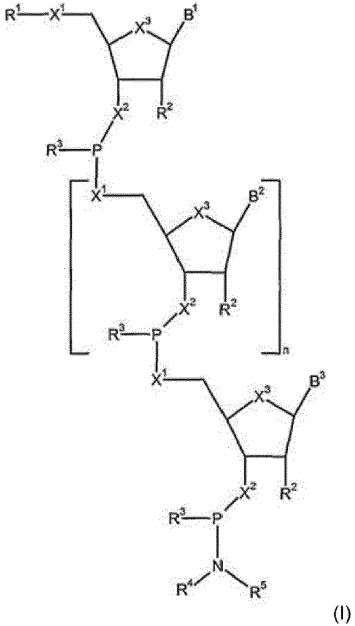 wherein,
X¹ is each independently -O- or -S-;
X² is each independently -O- or -S-;
X³ is each independently -O-, -S-, -CH₂- or -(CH₂)₂-;
R¹ is a protecting group;
R² is each independently -H, -NHR⁶, halogen, -CN, -CF₃, or a hydroxyl group protected by an acyl-based protecting group, an ether-based protecting group or a silyl-based protecting group;
R³ is each independently -OCH₂CH₂CN, -SCH₂CH₂CN, a substituted or unsubstituted aliphatic group, -OR⁷ or -SR⁷;
R⁴ and R⁵ are each independently a substituted or unsubstituted aliphatic group, a substituted or unsubstituted aromatic group, a substituted or unsubstituted aralkyl group; or
R⁴ and R⁵ taken together with nitrogen to which they are bound form a heterocycloalkyl group or a heteroaromatic group;
R⁶ is each independently -H, a substituted or unsubstituted aliphatic group, a substituted or unsubstituted aromatic group, a substituted or unsubstituted aralkyl group, or a protecting group;
R⁷ is each independently a substituted or unsubstituted aliphatic group, a substituted or unsubstituted aromatic group, or a substituted or unsubstituted aralkyl group;
B¹, B² and B³ are each independently -H, or a protected or unprotected base; and
n is 0 or a positive integer;
or a stereoisomer thereof.

9. The method according to claim 8, wherein n is 0 or 1.

10. The method according to claim 8 or 9, wherein R² is -H.

11. The method according to any one of claims 8 to 10, wherein R³ is - OCH₂CH₂CN.

12. The method according to any one of claims 1 to 11, wherein the nucleoside phosphoramidite having one or more nucleoside moieties and a linker moiety is a nucleoside phosphoramidite represented by the following formula (II): 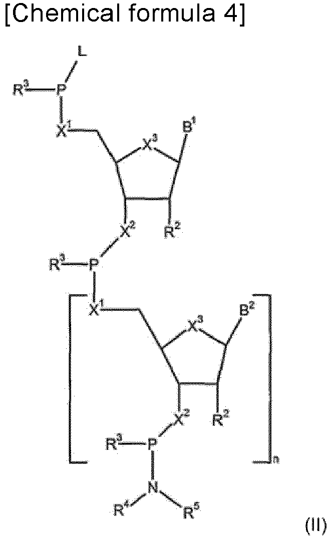 wherein,
X¹ is each independently -O- or -S-;
X² is each independently -O- or -S-;
X³ is each independently -O-, -S-, -CH₂- or -(CH₂)₂-;
L is a linker;
R² is each independently -H, -NHR⁶, halogen, -CN, -CF₃, or a hydroxyl group protected by an acyl-based protecting group, an ether-based protecting group or a silyl-based protecting group;
R³ is each independently -OCH₂CH₂CN, -SCH₂CH₂CN, a substituted or unsubstituted aliphatic group, -OR⁷ or -SR⁷;
R⁴ and R⁵ are each independently a substituted or unsubstituted aliphatic group, a substituted or unsubstituted aromatic group, a substituted or unsubstituted aralkyl group; or
R⁴ and R⁵ taken together with nitrogen to which they are bound form a heterocycloalkyl group or a heteroaromatic group;
R⁶ is each independently -H, a substituted or unsubstituted aliphatic group, a substituted or unsubstituted aromatic group, a substituted or unsubstituted aralkyl group, or a protecting group;
R⁷ is each independently a substituted or unsubstituted aliphatic group, a substituted or unsubstituted aromatic group, or a substituted or unsubstituted aralkyl group;
B¹ and B² are each independently -H, or a protected or unprotected base; and n is 0 or a positive integer;
or a stereoisomer thereof.

13. A method for producing an oligonucleotide, comprising performing one or more coupling steps of binding a nucleoside phosphoramidite to a 3' or 5' hydroxyl or thiol group of a nucleotide or nucleoside in the presence of an activator,
wherein in at least one coupling step, the nucleoside phosphoramidite is
(a) a nucleoside phosphoramidite having two or three nucleoside moieties, or
(b) a nucleoside phosphoramidite having one or more nucleoside moieties and a linker moiety, and
the activator has a structure represented by the following formula: 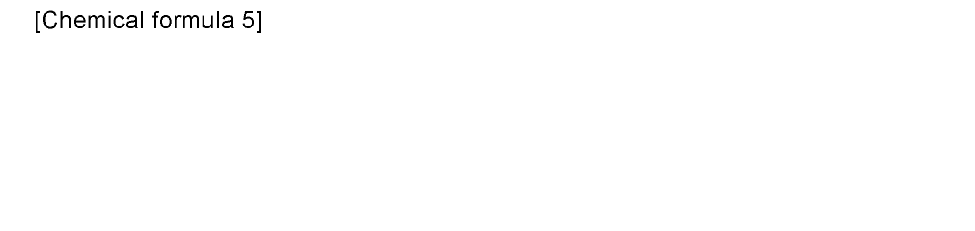 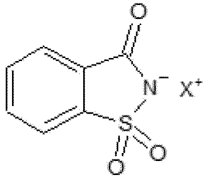
wherein X is an organic base,
or by the following formula: 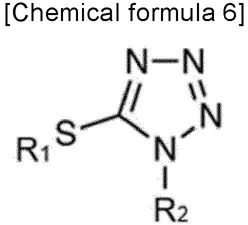
wherein R¹ and R² are each independently selected from the group consisting of H, linear or branched C₁₋₇ alkyl groups, and aromatic groups which may be optionally substituted.

14. The method according to claim 13, wherein
X is N-methylimidazole, pyridine or 3-methylpyridine,
R¹ is H, CₙH₂ₙ₊₁ or a benzyl group,
R² is H, CH₃, or C₆H₅, and
n is 1, 2 or 3.

15. A method for producing an oligonucleotide, comprising performing one or more coupling steps of binding a nucleoside phosphoramidite to a 3' or 5' hydroxyl or thiol group of a nucleotide or nucleoside in the presence of an activator,
wherein in at least one coupling step, the nucleoside phosphoramidite is
(a) a nucleoside phosphoramidite having two or more nucleoside moieties, or
(b) a nucleoside phosphoramidite having one or more nucleoside moieties and a linker moiety, and
the HOMO energy (a.u.) of the activator is -0.21407 to -0.16858 in acetonitrile, and
the orbital coefficient of the activator is 0.31531 to 0.59405 in acetonitrile.

16. The method according to claim 15, wherein the pKa of the activator is 3.65 to 7 in water.

17. A method for producing an oligonucleotide, comprising performing one or more coupling steps of binding a nucleoside phosphoramidite to a 3' or 5' hydroxyl or thiol group of a nucleotide or nucleoside in the presence of an activator,
wherein in at least one coupling step, the nucleoside phosphoramidite is
(a) a nucleoside phosphoramidite having two or three nucleoside moieties, or
(b) a nucleoside phosphoramidite having one or more nucleoside moieties and a linker moiety, and
the HOMO energy (a.u.) of the activator is -0.22024 to -0.16858 in acetonitrile, and
the orbital coefficient of the activator is 0.31531 to 0.59405 in acetonitrile.

18. The method according to claim 17, wherein the pKa of the activator is 3.65 to 7 in water.

19. A method for producing an oligonucleotide, comprising performing one or more coupling steps of binding a nucleoside phosphoramidite to a 3' or 5' hydroxyl or thiol group of a nucleotide or nucleoside in the presence of an activator,
wherein in at least one coupling step, the nucleoside phosphoramidite is
(a) a nucleoside phosphoramidite having two or three nucleoside moieties, or
(b) a nucleoside phosphoramidite having one or more nucleoside moieties and a linker moiety, and
the pKa of the activator is 3.65 to 7 in water.

20. A method for producing an oligonucleotide, comprising performing one or more coupling steps of binding a nucleoside phosphoramidite to a 3' or 5' hydroxyl or thiol group of a nucleotide or nucleoside in the presence of an activator,
wherein in at least one coupling step, the nucleoside phosphoramidite is
(a) a nucleoside phosphoramidite having two or more nucleoside moieties, or
(b) a nucleoside phosphoramidite having one or more nucleoside moieties and a linker moiety, and
the pKa of the activator is 4.3 to 7 in water.
